Europäisches Patentamt

European Patent Office

Office européen des brevets

(11) Publication number : **0 517 464 A1**

# EUROPEAN PATENT APPLICATION

(21) Application number : **92305000.9**

(22) Date of filing : **01.06.92**

(51) Int. Cl.$^5$ : **C07K 7/06,** C07K 1/02, C07K 1/04, A61K 37/02

(30) Priority : **03.06.91 US 708035**

(43) Date of publication of application :
**09.12.92 Bulletin 92/50**

(84) Designated Contracting States :
**AT BE CH DE DK ES FR GB GR IT LI LU NL PT SE**

(71) Applicant : **IMMUNOBIOLOGY RESEARCH INSTITUTE, INC.**
**Route 22 East P.O. Box 999**
**Annandale New Jersey 08801-0999 (US)**

(72) Inventor : **Goldstein, Gideon**
**30 Dorison Drive**
**Short Hills, N.J. 07078 (US)**
Inventor : **Audhya, Tapan**
**9 Argonne Farm Road**
**Bridgewater, N.J. 08807 (US)**
Inventor : **Heavner, George**
**Box 73B, Summer Road**
**Flemington, N.J. 08822 (US)**
Inventor : **Anwer, Mohmed K.**
**409 Willow Court**
**Flemington, N.J. 08822 (US)**

(74) Representative : **Fisher, Adrian John et al**
**CARPMAELS & RANSFORD 43 Bloomsbury Square**
**London WC1A 2RA (GB)**

(54) **Peptides useful in regulating the immune and nervous systems.**

㊼ Pentapeptides are disclosed which are capable of regulating the function of cells of the mammalian immune and/or nervous system. Also provided are pharmaceutical compositions containing the peptides and methods of use thereof.

EP 0 517 464 A1

Jouve, 18, rue Saint-Denis, 75001 PARIS

The present invention relates generally to peptides useful in the treatment of aging and abnormalities of the immune and central nervous system.

## Background of the Invention

The immunomodulatory protein thymopoietin has been isolated from bovine and human thymus. Additionally, small peptides have been chemically synthesized which mimic the biological activity of thymopoietin. See, e.g. U. S. Patent 4,505,853 and corresponding EP Application No. 146,266.

A large body of articles and patents have now been published relating to such proteins and synthesized peptides. U. S. Patent No. 4,190,646 discloses the pentapeptide thymopentin which is the active site of thymopoietin and has the sequence Arg-Lys-Asp-Val-Tyr, as well as peptide compositions in which various groups are substituted onto the amino and/or carboxyl termini of this pentapeptide.

Thymopoietin is known to regulate cholinergic neuromuscular transmission [G. Goldstein and W. H. Hoffman, J. Neurol. Neurosurg. Psychiatry, 31:453-459 (1968); and G. Goldstein, Nature, 247:11-14 (1974)]. This neuromuscular effect is caused by thymopoietin binding to the nicotinic Acetylcholine receptor, a ligand-regulated ion channel from the vertebrate neuromuscular junction and fish electric organ [F. Revah et al, Proc. Natl. Acad. Sci. USA, 84:3477-3481 (1987); and K. Venkatasubramanian et al, Proc. Natl. Acad. Sci. USA, 83:3171-3174 (1986)]. Thymopoietin is present within the brain, as are thymopoietin receptors (TPR), so that thymopoietin is almost certainly involved in brain function.

More recently, thymopentin has been identified as an antagonist of stress-induced changes, exhibiting stress-protective activity [V. Klusa et al, Regulatory Peptides, 27:355-365 (1990)].

There remains a need in the art for additional peptides as diagnostic and/or therapeutic agents which are useful in treating dysfunctions of the immune system in mammals, including those associated with aging and various physical conditions, as well as peptides useful for treating disorders in brain functions.

## Summary of the Invention

The present invention describes a series of novel pentapeptides capable of regulating the function of cells of the immune and central nervous system and characterized by unusually high activity as immunomodulators in comparison to known peptides.

As one aspect, the present invention relates to novel pentapeptides having the following formula:

$$R^1\text{-}V\text{-}W\text{-}X\text{-}Y\text{-}Z\text{-}R^2$$

or a pharmaceutically acceptable acid- or base-addition salt thereof, wherein $R^1$, V, W, X, Y, and Z are as defined below in the detailed description. The presence of a proline (or proline analog) in position 4 (or Y) of peptides of this invention confers upon the peptides an increased resistance to enzymes as compared to pentapeptides having similar amino acid compositions in position #1-3 and 5. Thus, the peptides of the invention have a greater in vivo potency upon oral administration as compared to the peptides of the prior art.

As another aspect, the present invention provides methods for preparing the above-described peptides by solution synthesis, solid-phase synthesis or enzymatic synthesis.

In yet another aspect, the present invention provides pharmaceutical compositions comprising one or more of the above-identified peptides in combination with a pharmaceutically acceptable carrier.

Still a further aspect of the present invention provides methods for treating a variety of disorders and deficiencies related to the immune system, in particular, the effects of aging caused by the shrinkage of the thymus gland over time, and psychiatric disorders such as anxiety and depression, comprising administering an effective amount of a pharmaceutical composition of this invention to an affected subject.

Other aspects and advantages of the present invention are disclosed in the following detailed description containing examples of presently preferred embodiments.

## Brief Description of the Drawings

Fig. 1 is a bar graph illustrating the enzymatic stability of thymopentin and Acetyl-Arg-Pro-Asp-Pro-Phe-$NH_2$ (peptide IRI-514) exposed to duodenum in a diffusion cell at 0, 2 and 4 hours as described in Example 19.

Fig. 2 shows a dose-response effect of orally-administered peptide IRI-514 on neuromuscular transmission, with a threshold dose of 0.05 mg/kg, as described in Example 18.

Fig. 3 summarizes four experiments evaluating the ability of cells derived from peptide IRI-514-treated and placebo-treated animals to generate T suppressor cells, as described in Example 19.

Fig. 4 illustrates the effects of peptide IRI-514, vehicle treated, and control (unstressed) on behavioral response after social stress-induced anxiety in rats, described in Example 20.

Detailed Description of the Invention

The present invention provides peptides capable of regulating and affecting the mammalian immune and/or central nervous system. These pentapeptides have been demonstrated to have biological activity in the neuromuscular assay, in enhancing anti-CD3 stimulated development of T suppressor cells, in attenuating stress induced elevation of HPA axis hormones, and in preventing stress induced behavioral changes. These pentapeptides are further characterized by surprising potency in oral administration in contrast to other known peptides.

The present invention provides a series of novel pentapeptides having the following formula:

$$R^1-V-W-X-Y-Z-R^2$$

or a pharmaceutically acceptable acid- or base-addition salt thereof, wherein $R^1$ is hydrogen or a $C_1$ to $C_{10}$ lower alkyl or alkanoyl;

V is Arg;

W is Pro, dehydro-Pro or hydroxy-Pro;

X is Asp, Ser, Thr, Ala, Asn, Glu, or Gln;

Y is Pro, dehydro-Pro, or hydroxy-Pro;

Z is Phe or Tyr, optionally substituted with one or more halogen, nitro or hydroxyl group;

and

$R^2$ is OH or $NR^3R^4$,

wherein $R^3$ and $R^4$ are each independently selected from the group consisting of H, a straight chain or branched alkyl or alkenyl having 1 to 6 carbon atoms, optionally substituted with an aryl group or aryl substituted with either a halogen or a straight chain, a branched alkyl or alkenyl having 1 to 6 carbon atoms, or $R^3$ and $R^4$ together comprise a cyclic methylene group of 3 to 7 carbon atoms, and

wherein optionally one or more of V, W, X, Y and Z is a D amino acid.

Throughout this disclosure, the amino acid components of the peptides and certain materials used in their preparation are identified by abbreviations for convenience. Most of the three letter abbreviations for amino acids are well known. As above indicated, all amino acids in the above formula are normally in the L-isomeric configuration; however in any particular peptide one or more amino acid may be in the D-isomeric configuration.

Peptides of these formulae, having a Pro or analog thereof in amino acid position 2 and in amino acid position 4 are characterized by enhanced stability and resistance to attack by endo- and exopeptidases and trypsin-like enzymes in the digestive tract and in serum. This enhanced resistance to enzymatic digestion in the intestinal tract makes such peptides particularly well suited to oral administration, providing the peptides with surprising potency compared to other known peptides. Because these peptides are extremely active in oral dosages, they are especially well suited for a pharmaceutical preparation in tablet form.

One preferred pentapeptide is Acetyl-Arg-Pro-Asp-Pro-Z-NH$_2$, where Z may be Tyr or the optionally-substituted Phe selections. A presently most preferred pentapeptide is Acetyl-Arg-Pro-Asp-Pro-Phe-NH$_2$, called IRI-514. Surprisingly, this peptide has high oral potency.

Particularly preferred peptides of this invention, therefore, include:

Acetyl-Arg-Pro-Asp-Pro-Phe-NH$_2$
Acetyl-Arg-Pro-Asp-Pro-Tyr-NH$_2$
Acetyl-Arg-Pro-Asn-Pro-Phe-NH$_2$
Acetyl-Arg-Pro-Asn-Pro-Tyr-NH$_2$
Acetyl-Arg-Pro-Ala-Pro-Phe-NH$_2$
Acetyl-Arg-Pro-Ala-Pro-Tyr-NH$_2$
Acetyl-Arg-Pro-Glu-Pro-Tyr-NH$_2$
Acetyl-Arg-Pro-Ser-Pro-Phe-NH$_2$
Acetyl-Arg-Pro-Thr-Pro-Phe-NH$_2$
Acetyl-Arg-Pro-Thr-Pro-Tyr-NH$_2$
Acetyl-Arg-Pro-Ser-Pro-Tyr-NH$_2$
Acetyl-Arg-Pro-Asp-Pro-pClPhe-NH$_2$
Decanoyl-Arg-Pro-Asp-Pro-Phe-NH$_2$
Acetyl-Arg-Pro-Glu-Pro-Phe-NH$_2$
Butyryl-Arg-Pro-Asn-Pro-Phe-NH$_2$

Although well adapted for oral therapies, the peptides according to the invention may also be administered by any suitable route, e.g. by injection or topically. For use in topical preparations, preferably, decanoyl is substituted for Acetyl at position $R^1$ because its lipophilic properties make it particularly well suited for absorption into the skin.

The peptides of this invention may generally be prepared following known techniques. Conveniently, synthetic production of the polypeptide of the invention may be according to the solid phase synthetic method described by Merrifield in J.A.C.S, 85: 2149-2154 (1963). This technique is well understood and is a common method for preparation of peptides. The solid phase method of synthesis involves the stepwise addition of protected amino acids to a growing peptide chain which is bound by covalent bonds to a solid resin particle. By this procedure, reagents and by-products are removed by filtration, thus eliminating the necessity of purifying intermediates. The general concept of this method depends on attachment of the first amino acid of the chain to a solid polymer by a covalent bond. Succeeding protected amino acids are added, one at a time (stepwise strategy), or in blocks (segment strategy), until the desired sequence is assembled. Finally, the protected peptide is removed from the solid resin support and the protecting groups are cleaved off.

The amino acids may be attached to any suitable polymer as a resin. The resin must contain a functional group to which the first protected amino acid can be firmly linked by a covalent bond. Various polymers are suitable for this purpose, such as cellulose, polyvinyl alcohol, polymethylmethacrylate, and polystyrene. Appropriate protective groups usable in such synthesis include t-butyloxycarbonyl (Boc), benzyl (Bzl), t-amyloxycarbonyl (Aoc), tosyl (Tos), o-bromophenylmethoxycarbonyl (BrZ), 2,6-dichlorobenzyl (BzlCl$_2$), and phenylmethoxycarbonyl (Z or CBZ). Additional protective groups are identified in Merrifield, cited above, as well as in J.F.W. McOmie, "Protective Groups in Organic Chemistry", Plenum Press, New York, 1973. Both of these texts are incorporated herein by reference.

The general procedure of preparation of the peptides of this invention involves initially attaching the protected carboxyl-terminal amino acid to the resin. After attachment the resin is filtered, washed and the protecting group (desirably t-butyloxycarbonyl) on the alpha amino group of the carboxyl-terminal amino acid is removed. The removal of this protecting group must take place, of course, without breaking the bond between that amino acid and the resin. The next amino, and if necessary, side chain protected amino acid, is then coupled to the free a-amino group of the amino acid on the resin. This coupling takes place by the formation of an amide bond between the free carboxyl group of the second amino acid and the amino group of the first amino acid attached to the resin. This sequence of events is repeated with successive amino acids until all amino acids are attached to the resin. Finally, the protected peptide is cleaved from the resin and the protecting groups removed to reveal the desired peptide. The cleavage techniques used to separate the peptide from the resin and to remove the protecting groups depend upon the selection of resin and protecting groups and are known to those familiar with the art of peptide synthesis.

Alternative techniques for peptide synthesis are described in Bodanszky et al, Peptide Synthesis, 2nd edition (John Wiley and Sons: 1976). For example, the peptides of the invention may also be synthesized using standard solution peptide synthesis methodologies, involving either stepwise or block coupling of amino acids or peptide fragments using chemical or enzymatic methods of amide bond formation. [See, e.g. H.D. Jakubke in The Peptides, Analysis, Synthesis, Biology, Academic Press (New York 1987), p. 103-165; J.D. Glass, ibid., pp. 167-184; and European Patent 0324659 A2, describing enzymatic peptide synthesis methods.] These solution synthesis methods are well known in the art.

The peptides of this invention may also be produced by other techniques known to those of skill in the art, for example, genetic engineering techniques. See, e.g., Sambrook et al, in Molecular Cloning, a Laboratory Manual, 2nd edition, Cold Spring Harbor Laboratory, Cold Spring Harbor, New York (1989).

The acid- or base-addition salts of these peptides are also disclosed by this invention for use as diagnostic and/or therapeutic agents. Acids which are able to form salts with these peptides include, but are not limited to, inorganic acids, such as hydrochloric acid, hydrobromic acid, perchloric acid, nitric acid, thiocyanic acid, sulfuric acid, phosphoric acid, and the like. Organic acids may also be employed to form the salts of the invention, e.g., formic acid, acetic acid, propionic acid, glycolic acid, lactic acid, pyruvic acid, oxalic acid, malonic acid, succinic acid, maleic acid, fumaric acid, citric acid, succinamic acid, anthranilic acid, cinnamic acid, naphthalene sulfonic acid, sulfanilic acid, and the like.

A nonexclusive list of bases which are able to form salts with those peptides having acidic moieties includes inorganic bases, such as sodium hydroxide, ammonium hydroxide, potassium hydroxide, and the like. Organic bases for such use include, without limitation thereto, mono-, di-, and tri-alkyl and aryl amines (e.g., triethylamine, diisopropylamine, methylamine, dimethylamine) and optionally substituted ethanolamines (e.g., ethanolamine, diethanolamine).

These peptides and compositions containing these peptides surprisingly demonstrate a variety of regulatory effects on the mammalian immune and/or central nervous system. For example, peptides of this invention offer treatment therapies for autoimmune disorders and aging, as well as other conditions characterized by a disorder of the immune system.

Because of the immunomodulatory characteristics of the subject peptides, they are therapeutically useful in the treatment of humans, and possibly animals, since they are capable of effecting changes in the immune system of the mammal.

The peptides of the invention may also be useful as anxiolytic therapeutic agents. For example, pretreatment of a patient with a peptide of the invention may reduce the levels of corticotropin releasing factor (CRF), which mediates stress reactions. Thus, such peptides are useful as anti-depressive treatments, and similarly useful in treatment of other stress-induced disorders.

Also, the peptides according to the present invention may be used to diminish the effects of aging on the immune system. As the thymus shrinks with age, the level of thymopoietin, which is a thymus-derived polypeptide, decreases, and as a result stress-induced levels of CRF, adrenocorticotropic hormone (ACTH) and corticosteroids increase proportionally. Thus, administration of peptides of this invention which have biological activity similar to thymopoietin can help reduce the effects of aging related to inefficient or non-functioning immune systems.

The invention further provides pharmaceutical compositions containing one or more of the above-described peptides or acid- or base-addition salts thereof. The subject peptides or pharmaceutical compositions containing the peptides or their acid or basic salts are generally considered to be useful in any area in which cellular immunity is an issue and particularly where there are deficiencies in immunity.

The invention also provides a method for treatment of conditions resulting from disorder of the immune system and/or nervous system of a subject, which comprises administering to said subject a therapeutically-effective amount of at least one of the peptides or pharmaceutical compositions of this invention. As used herein, the term "therapeutically-effective amount" means an amount which is effective to treat the conditions referred to above.

To prepare the pharmaceutical compositions of the present invention, a peptide of this invention is combined as the active ingredient in intimate admixture with a pharmaceutical carrier according to conventional pharmaceutical compounding techniques. This carrier may take a wide variety of forms depending on the form of preparation desired for administration, e.g., oral, sublingual, rectal, nasal, or parenteral.

In preparing the compositions in the preferred oral dosage form, any of the usual pharmaceutical media may be employed. For oral liquid preparations (e.g., suspensions, elixirs, and solutions), media containing, for example, water, oils, alcohols, flavoring agents, preservatives, coloring agents and the like may be used. Carriers such as starches, sugars, diluents, granulating agents, lubricants, binders, disintegrating agents, and the like may be used to prepare oral solids (e.g., powders, capsules, and tablets). Controlled release forms may also be used. Because of their ease in administration, tablets and capsules represent the most advantageous oral dosage unit form, in which case solid pharmaceutical carriers are obviously employed. If desired, tablets may be sugar coated or enteric coated by standard techniques.

For parenteral products the carrier will usually comprise sterile water, although other ingredients may be included, e.g., to aid solubility or for preservation purposes. Injectable suspensions may also be prepared, in which case appropriate liquid carriers, suspending agents, and the like may be employed.

A pentapeptide of the present invention is generally active when parenterally administered in amounts above about 0.5 mg/kg of body weight to about 10 mg/kg body weight.

These peptides are more potent when administered orally than when administered parenterally. It is theorized that this occurs because the peptide is absorbed from the gut into the blood stream over a longer period of time, approximately 6 to about 8 hours. During this absorption period about 30% of the peptide is effectively absorbed. In contrast, once in the blood stream, drugs have short half life because they are cleared and then excreted by the kidneys. Prior art peptides have been observed to have a net absorption of approximately 15%. Peptides administered parenterally are cleared from the body much more quickly than drugs administered orally.

The peptides of the present invention are generally active when orally administered in amounts of between about 0.02 mg/kg of body weight to about 10 mg/kg of body weight, and preferably at about 1 mg/kg. Activity at this level makes these peptides particularly well adapted for pharmaceutical formulations in tablet size for oral administration.

The following examples are presented to illustrate the invention without specifically limiting the invention thereto. In the examples and throughout the specification, parts are by weight unless otherwise indicated. The examples employ the following abbreviations: TFA for trifluoroacetic acid; HOAc for acetic acid; $CH_2Cl_2$ for methylene chloride; $CH_3CN$ for acetonitrile; DMF for dimethyl formamide; $NH_4OAc$ for ammonium acetate; $NH_4OH$ for ammonium hydroxide; n-PrOH for n-propanol; n-BuOH for n-butanol; Pyr for pyridine; DCC for dicyclohexylcarbodiimide; HOBt for 1-hydroxy-benzotriazole; DMAP for dimethylaminopyridine; HF for hydrogen fluoride; TCA for trichloroacetic acid; BHA for benzhydrylamine resin; p-MBHA for p-methylbenzhydrylamine resin and MeOH for methanol. Other standard abbreviations can be identified by reference to The Peptides, Analysis, Synthesis, Biology, Vol. 1 and 2, ed. E. Gross and J. Meienhofer, Academic Press (New York 1987) and "IUPAC-IUB Commission on Biochemical Nomenclature", J. Biol. Chem., 242:6489-6497 (1970) and J. Biol. Chem., 250:3215-3216 (1975). The symbol Asx is used to indicate the presence of Asn, which is detected after analysis as Asp.

## Example 1 - Synthesis of Acetyl-Arg-Pro-Asp-Pro-Phe-NH$_2$

The pentapeptide amide was synthesized using the symmetrical anhydride coupling technique, except for arginine which was coupled via the DCC-HOBt method using the standard coupling protocols (Std 1 cycle, version 1.40) on the Applied Biosystems ABI 430A peptide synthesizer. The synthesis was initiated with 0.45 mmol of p-methylbenzhydrylamine resin.HCl (0.710 g., 0.64 mmol/g resin). The N- terminal Boc-group was removed by the end-NH2 program (version 1.40). The resulting peptide was Acetylated using acetic anhydride (1.0 mL) in CH$_2$Cl$_2$ (9 mL) containing 4-dimethylaminopyridine (15 mg) for 30 minutes. The resin was then washed with DMF(5X10 mL) and CH$_2$Cl$_2$(5X10 mL), and finally dried in a vacuum oven at 40°C (1.073 g).

The peptide was cleaved from the resin support (1.07 g, 0.45 mmol) by stirring in liquid HF (10 mL), m-methylanisole (1 mL), and dimethylsulfide (1 mL) for 1 hour at 0°C. After removal of excess HF under reduced pressure, the resin-peptide mixture was extracted with ether (3X50 mL). The ether extracts were discarded. The released peptide was then extracted with 20% aqueous HOAc (3X33 mL). After removal of solvents under reduced pressure, the residue obtained was dissolved in H$_2$O (40 mL) and lyophilized (0.248 g). This solid was dissolved in H$_2$O (10 mL) and passed through an Amberlite IRA-68 acetate form ion exchange column (60 g, 1.6 meq/mL, 2.73 cm i.d. X 18 cm length) in H$_2$O at a flow rate of 60 mL/h. The appropriate fractions were combined and lyophilized (0.240 g).

The crude peptide (0.240 g dissolved in 4 mL of buffer "A", see below) was purified by preparative RP-HPLC using a Vydac 218TP1022 column (22X250 mm). The mobile phases employed were as shown below:
        A=0.1% TFA/H$_2$O
        B=0.1% TFA/CH$_3$CN - H$_2$O 4:1 v/v
A linear gradient of 5% B to 23% B over 80 minutes at a flow rate of 14 mL/min. was used. The relevant fractions were combined and the organic solvents removed under reduced pressure. The aqueous residue was lyophilized (206 mg).

Thin layer chromatography (TLC) was performed on Merck F-254 silica plates (5X10 cm) in the following solvent systems (v/v):
        Rf(1) = 0.17 (1-BuOH:HOAc:H$_2$O, 4:1:1)
        Rf(2) = 0.47 (1-BuOH:HOAc:EtOAc:H$_2$O, 1:1:1:1)
        Rf(3) = 0.65 (1-BuOH:HOAc:Pyr:H$_2$O, 5:4:4:2)
Amino Acid Analysis (AAA):
The yields [found (expected)] were Arg 1.02(1), Pro 1.98(2), Asp 0.99(1), Phe 1.02(1), NH$_3$ 1.04(1). Fast Atom Bombardment/Mass Spectrometry (FAB-MS): [MH$^+$] at m/z 672 a.m.u. (Mol. Wt. 671.76), where MH$^+$ represents a positively charged mass ion; m/z is mass/charge; and a.m.u. is atomic mass units.

## Example 2 - Synthesis of Acetyl-Arg-Pro-Asp-Pro-Tyr-NH$_2$

The pentapeptide amide was synthesized as described in Example 1, with the substition of Tyr in the 5th position in place of Phe.
        TLC: Rf(1) = 0.20, Rf(2) = 0.33, Rf(3) = 0.85.
        AAA: Arg 1.06(1), Pro 1.99(2), Asp 1.03(1), Tyr 0.92(1), NH$_3$ 1.19(1)
        FAB-MS: [MH$^+$] at m/z 688 a.m.u. (Mol. Wt. 687.76).

## Example 3 - Synthesis of Acetyl-Arg-Pro-Asn-Pro-Phe-NH$_2$

The pentapeptide amide was synthesized as described in Example 1, with the substition of Asn for Asp in position 3. The characteristics of the peptide are as follows.
        TLC: Rf(1) = 0.22, Rf(2) = 0.56, Rf(3) = 0.72.
        AAA: Arg 1.02(1), Pro 1.97(2), Asx 1.01(1), Phe 1.01(1), NH$_3$ 1.95(2).
        FAB-MS: [MH$^+$] at m/z 671 a.m.u. (Mol. Wt. 670.78).

## Example 4 - Synthesis of Acetyl-Arg-Pro-Asn-Pro-Tyr-NH$_2$

The pentapeptide amide was synthesized as described in Example 1, with the substition of Asn for Asp in position 3 and Tyr for Phe in position 5. The characteristics of the peptide are as follows.

TLC: Rf(1) = 0.2, Rf(2) = 0.36, Rf(3) = 0.83.
AAA: Arg 1.07(1), Pro 1.98(2), Asx 1.04(1), Tyr 0.91(1), $NH_3$ 1.92(2).
FAB-MS: [$MH^+$] at m/z 687 a.m.u. (Mol. Wt. 686.77).

Example 5 - Synthesis of Acetyl-Arg-Pro-Ala-Pro-Phe-$NH_2$

The pentapeptide amide was synthesized as described in Example 1, with the substitution of Ala for Asp in position 3. The characteristics of the peptide are as follows.
TLC: Rf(1) = 0.18, Rf(2) = 0.49, Rf(3) = 0.69.
AAA: Arg 0.98(1), Pro 1.97(2), Ala 1.03(1), Phe 1.02(1), $NH_3$ 1.00(1).
FAB-MS: [$MH^+$] at m/z 628 a.m.u. (Mol. Wt. 627.75).

Example 6 - Synthesis of Acetyl-Arg-Pro-Ala-Pro-Tyr-$NH_2$

The pentapeptide amide was synthesized as described in Example 1, with the substitution of Ala for Asp in position 3 and Tyr for Phe in position 5. The characteristics of the peptide are as follows.
TLC: Rf(1) = 0.16, Rf(2) = 0.32, Rf(3) = 0.71.
AAA: Arg 1.04(1), Pro 1.96(2), Ala 0.99(1), Tyr 1.02(1), $NH_3$ 1.03(1).
FAB-MS: [$MH^+$] at m/z 644 a.m.u. (Mol. Wt. 643.75).

Example 7 - Synthesis of Acetyl-Arg-Pro-Glu-Pro-Phe-$NH_2$

The pentapeptide amide was synthesized as described in Example 1, with the substitution of Glu for Asp in position 3. The characteristics of the peptide are as follows.
TLC: Rf(1) = 0.26, Rf(2) = 0.58, Rf(3) = 0.74.
AAA: Arg 0.97(1), Pro 2.03(2), Glu 1.01(1), Phe 1.00(1), $NH_3$ 1.02(1).
FAB-MS: [$MH^+$] at m/z 686 a.m.u. (Mol. Wt. 685.79).

Example 8 - Synthesis of Acetyl-Arg-Pro-Glu-Pro-Tyr-$NH_2$

The pentapeptide amide was synthesized as described in Example 1, with the substitution of Glu for Asp in position 3 and Tyr for Phe in position 5. The characteristics of the peptide are as follows.
TLC: Rf(1) = 0.18, Rf(2) = 0.35, Rf(3) = 0.66.
AAA: Arg 1.02(1), Pro 1.95(2), Glu 1.03(1), Tyr 1.01(1), $NH_3$ 0.99(1).
FAB-MS: [$MH^+$] at m/z 702 a.m.u. (Mol. Wt. 701.79).

Example 9 - Synthesis of Acetyl-Arg-Pro-Ser-Pro-Phe-$NH_2$

The pentapeptide amide was synthesized as described in Example 1, with the substitution of Ser for Asp in position 3. The characteristics of the peptide are as follows.
TLC: Rf(1) = 0.28, Rf(2) = 0.62, Rf(3) = 0.78.
AAA: Arg 1.01(1), Pro 2.00(2), Ser 0.62(1), Phe 0.99(1), $NH_3$ 1.16(1).
FAB-MS: [$MH^+$] at m/z 644 a.m.u. (Mol. Wt. 643.75).

Example 10 - Synthesis of Acetyl-Arg-Pro-Thr-Pro-Phe-$NH_2$

The pentapeptide amide was synthesized as described in Example 1, with the substitution of Thr for Asp in position 3. The characteristics of the peptide are as follows.
TLC: Rf(1) = 0.13, Rf(2) - 0.47, Rf(3) = 0.72.
AAA: Arg 1.00(1), Pro 2.00(2), Thr 0.82(1), Phe 1.15(1), $NH_3$ 1.19(1).
FAB-MS: [$MH^+$] at m/z 658 a.m.u. (Mol. Wt. 657.78).

Example 11 - Synthesis of Acetyl-Arg-Pro-Thr-Pro-Tyr-$NH_2$

The pentapeptide amide was synthesized as described in Example 1, with the substitution of Thr for Asp in position 3 and Tyr for Phe in position 5. The characteristics of the peptide are as follows.
TLC: Rf(1) = 0.13, Rf(2) = 0.43, Rf(3) = 0.66.
AAA: Arg 1.00(1), Pro 2.00(2), Thr 0.87(1), Phe 1.00(1), $NH_3$ 0.93(1).

FAB-MS: [MH+] at m/z 674 a.m.u. (Mol. Wt. 673.78).

## Example 12 - Synthesis of Acetyl-Arg-Pro-Ser-Pro-Tyr-NH$_2$

The pentapeptide amide was synthesized as described in Example 1, with the substitution of Ser for Asp in position 3 and Tyr for Phe in position 5. The characteristics of the peptide are as follows.
TLC: Rf(1) = 0.29, Rf(2) = 0.65, Rf(3) = 0.82.
AAA: Arg 1.03(1), Pro 1.95(2), Ser 0.65(1), Tyr 1.01(1), NH$_3$ 1.20(1).
FAB-MS: [MH+] at m/z 660 a.m.u. (Mol. Wt. 659.75).

## Example 13 - Synthesis of Acetyl-Arg-Pro-Asp-Pro-pClPhe-NH$_2$

The pentapeptide amide was synthesized as described in Example 1, with the substitution of parachloro-Phe for Phe in position 5. The characteristics of the peptide are as follows.
TLC: Rf(1) = 0.19, Rf(2) = 0.49, Rf(3) = 0.66.
AAA: Arg 1.00(1), Pro 1.99(2), Asp 0.97(1), pClPhe 1.04(1), NH$_3$ 1.02(1).
FAB-MS: [MH+] at m/z 706 a.m.u. (Mol. Wt. 706.29).

## Example 14 - Synthesis of Butyryl-Arg-Pro-Asn-Pro-Phe-NH$_2$

The pentapeptide amide was synthesized as described in Example 1, with the substitution of Asn for Asp in position 3. The N-terminal Boc-group was removed by the end-NH$_2$ program. The N-terminal butyryl-group was introduced by coupling with n-butyric acid using diisopropyl carbodiimide and HOBt. The characteristics of the peptide are as follows.
TLC: Rf(1) = 0.32, Rf(2) = 0.65, Rf(3) = 0.84.
AAA: Arg 1.01(1), Pro 1.98(2), Asx 1.02(1), Phe 1.00(1), NH$_3$ 1.98(2).
FAB-MS: [MH+] at m/z 699 a.m.u. (Mol. Wt. 698.83).

## Example 15 - Synthesis of Decanoyl-Arg-Pro-Asp-Pro-Phe-NH$_2$

The pentapeptide amide was synthesized substantially as described in Example 1, with the following modifications. The solid-phase peptide synthesis was initiated with 5 mmoles of benzhydrylamine resin and after the sequential introduction of the amino acids, the N-terminal Boc-group was removed. The resulting peptide-resin was treated with decanoic acid in DMF containing hydroxy benzotriazole and diisopropylcarbodiimide. The resin was then washed and dried (11.17 g).

The peptide was cleaved from the resin support using liquid HF as described in Example 1. The crude peptide (1.8 g), after ion-exchange, was purified by preparative RP-HPLC using the same mobile phases described in Example 1. The gradient used was 25% B to 50% B over 100 minutes at a flow rate of 14 mL/min. The yield of the pure product was 1.19 g. The characteristics of this peptide are as follows:
TLC: Rf(1) = 0.30, Rf(2) = 0.64, Rf(3) = 0.82.
AAA: Arg 0.99(1), Pro 2.00(2), Asp 0.99(1), Phe 1.01(1), NH$_3$ 1.07(1).
FAB-MS: [MH+] at m/z 785 a.m.u. (Mol. Wt. 784.72).

## Example 16 - Solution-phase Synthesis of Acetyl-Arg-Pro-Asp-Pro-Phe-NH$_2$

This peptide was also made by solution synthesis as described below.

## A. Boc-Pro-Phe-NH$_2$:

To a solution of Boc-Pro-OH (4.304 g, 20 mmoles) in ethyl acetate (200 mL) at -15°C, N-methylmorpholine (2.2 mL, 20 mmoles) and isobutyl chloroformate (2.6 mL, 20 mmoles) were added. After an activation time of 10 minutes at -15°C, a solution of HCl.Phe-NH$_2$ (4.014 g, 20 mmoles) and N-methylmorpholine (2.2 mL, 20 mmoles) in dimethylformamide (20 mL) was added. The reaction mixture was stirred at -15°C for 30 minutes and allowed to warm to room temperature. After stirring for 2 hours at room temperature, the reaction mixture was quenched with 10% potassium carbonate solution (30 mL). The two clear layers were transferred to a separatory funnel, and the organic layer was washed successively with 10% potassium carbonate solution (2X30 mL), saturated sodium chloride solution (1X30 mL), 1N HCl (2X30 mL) and saturated sodium chloride solution (2X30 mL). After drying over anhydrous sodium sulfate, the organic solution was filtered and concentrated on a rotary evaporator under house vacuum. The product was precipitated with hexane, filtered and dried.

Yield (Theoretical): 7.229 g.

Experimental Yield: 6.795 g. (94% of Theory)

B. HCl.Pro-Phe-NH$_2$:

To a solution of Boc-Pro-Phe-NH$_2$ prepared as described in A above (6.506 g, 18 mmoles) in ethyl acetate (50 mL) and glacial acetic acid (25 mL), 5N HCl-ethyl acetate (20 mL) and anisole (2 mL) were added. The solution was stirred for 1 hour and concentrated on a rotary evaporator. The hydrochloride salt of the dipeptide was precipitated by the addition of ether (200 mL). The solid was filtered and dried in vacuum over KOH pellets.

C. Boc-Asp(OBzl)-Pro-Phe-NH$_2$:

The mixed anhydride prepared by reacting Boc-Asp(OBzl) (5.82 g, 18 mmoles), N-methylmorpholine (1.98 mL, 18 mmoles) and isobutyl chloroformate (2.34 mL, 18 mmoles) in ethyl acetate (200 mL) at -15°C, was treated with the solution of HCl.Pro-Phe-NH$_2$ (prepared as described in B above) and N-methylmorpholine (1.98 mL, 18 mmoles) in dimethylformamide (40 mL). The reaction was allowed to proceed for 30 minutes at -15°C and 2 hours at room temperature. The product was worked up as described earlier and reprecipitated from ethyl acetate-hexane.

Yield (Theoretical): 10.20 g.

Experimental Yield: 9.76 g (95.7% of Theory).

D. Boc-Pro-Asp(OBzl)-Pro-Phe-NH$_2$:

Boc-Asp(OBzl)-Pro-Phe-NH$_2$ prepared as described in C above (9.067 g, 16 mmoles) was deprotected using HCl-ethyl acetate as described above in part B and the hydrochloride salt of the tripeptide was precipitated with ether, filtered and dried.

The mixed anhydride prepared from Boc-Pro (3.44 g, 16 mmoles), N-methyl morpholine (1.76 mL, 16 mmoles) and isobutylchloroformate (2.08 mL, 16 mmoles) in ethyl acetate (160 mL) was treated with the solution of HCl.Asp(OBzl)-Pro-Phe-NH$_2$ and N-methyl morpholine (1.76 mL, 16 mmoles) in dimethylformamide (30 mL). The reaction conditions and the product work up were described earlier. The product was crystallized from ethyl acetate-hexane.

Yield (Theoretical): 10.62 g.

Experimental Yield: 10.09 g (95% of Theory).

E. Z-Arg-Pro-Asp(OBzl)-Pro-Phe-NH$_2$:

Boc-Pro-Asp(OBzl)-Pro-Phe-NH$_2$ prepared as described in D above (9.96 g, 15 mmoles) was deprotected using HCl-ethyl acetate and the hydrochloride salt of the tetrapeptide was precipitated with ether, filtered and dried.

To a stirred solution of Z-Arg.HCl (5.17 g, 15 mmoles) in dimethylformamide (25 mL) at 25°C was added a solution of HCl.Pro-Asp(OBzl)-Pro-Phe-NH$_2$ and N-methylmorpholine (1.65 mL, 15 mmoles) in dimethylformamide (25 mL). To the resulting solution, diethyl cyanophosphonate (2.45 g, 15 mmoles) and N-methylmorpholine (1.65 mL, 15 mmoles) were added and the mixture stirred for 4 hours. The reaction was quenched by the addition of 10% aqueous acetic acid (10 mL) and the solvents were removed on a rotary evaporator under reduced pressure. The resulting solid was triturated with 10% potassium carbonate and then with water and filtered.

Yield (Theoretical): 12.81 g.

Experimental Yield: 10.25 g (80% of Theory).

F. Acetyl-Arg-Pro-Asp-Pro-Phe-NH$_2$[SEQ ID NO:1]:

To a solution of the Z-Arg-Pro-Asp(OBzl)-Pro-Phe-NH$_2$ prepared as described in E above (11.10 g, 13 mmoles) in acetic acid (50 mL), 20% Pd hydroxide on carbon (1.0 g) was added and subjected to hydrogenation at 45 psi of hydrogen gas. The reaction was monitored by TLC, and after 1 hour the catalyst was filtered off and the solvents were removed under reduced pressure, resulting in Arg-Pro-Asp-Pro-Phe-NH$_2$. The residue was dissolved in water (50 mL) and freeze-dried.

Arg-Pro-Asp-Pro-Phe-NH$_2$ was dissolved in acetic acid (20 mL) and treated with acetic anhydride (6.12 g, 60 mmoles) with constant stirring. After 1 hour, the reaction was quenched by the addition of water (20 mL) and the solvents removed under reduced pressure. The residue was dissolved in water (50 mL) and freeze-dried.

The crude peptide thus obtained was purified by preparative RP-HPLC following the procedure described under Example 1. The product was lyophilized to constant weight. (Peptide content: 90%).

Yield (Theoretical): 8.73 g. Experimental Yield: 7.56 g (77.9% of Theory). The compound was indistinguishable from the product obtained in Example 1.

Example 17 - Enzymatic Stability

To illustrate the stability of a peptide of the present invention the following assay was performed.

Thymopentin and the peptide Acetyl-Arg-Pro-Asp-Pro-Phe-NH$_2$, identified as IRI-514, were dissolved separately in phosphate buffer (pH 5.5). A rat duodenum was opened and attached to a 3.5 mL diffusion cell [Crownglass, NJ] with buffer in both chambers. The selected test peptide in phosphate buffer (pH 5.5) was applied to both the cells to a final concentration of 1 mg/mL, and samples were taken at 0, 2 and 4 hours. The aliquots were subjected to high performance liquid chromatography (HPLC) with ultraviolet detection at 220 nm. The solvent system for thymopentin was phosphate buffer and methanol. For IRI-514, acetonitrile and phosphate buffer were used as the solvent system.

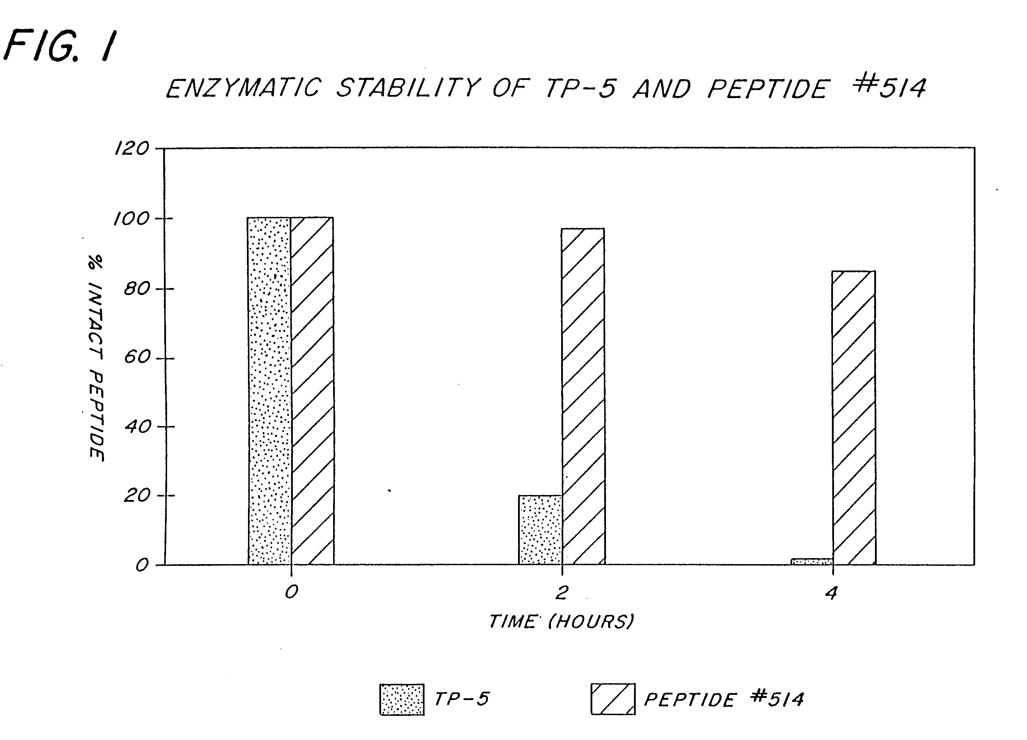

The bar graph of Fig. 1 illustrates that at 0 hours, thymopentin (TP-5) and IRI-514 were 100% intact. After 2 hours IRI-514 was still approximately 100% intact while only 20% of TP-5 remained intact. Finally, at 4 hours, the amount of intact IRI-514 is approximately 90%, while the amount of intact TP-5 was negligible. Furthermore, the inventors postulate that the decrease in intact IRI-514 after 4 hours may be due to absorption to duodenum tissue since no degradation peaks were detected by HPLC.

Example 18 - Neuromuscular Assay

This assay measures the ability of a peptide to affect cholinergic transmission at the neuromuscular junction. Thymopoietin and thymopentin are known to cause this effect on neuromuscular transmission.

Various doses of the test peptide are dissolved in phosphate buffered saline and injected subcutaneously or administered orally into female mice, weighing 25-30 grams (CD1 strain). The electromyographic assay is performed according to G. Goldstein et al, J. Neurol. Neurosurg. Psychiat., 31:453-459 (1968), 24 or 48 hours after administration of the peptide. The mice are anaesthetized with 0.5 mL of a 10% urethane solution. The nerve is stimulated with a Grass S-48 stimulator and a Grass SIU-5A stimulus isolation unit (Grass medical instruments, Quincy, MA) and the electromyographic response is recorded with a Tektronix storage oscilloscope-5111 coupled to a 5A21N differential amplifier and a 5B10N time base (Tektronix, Beaverton, OR).

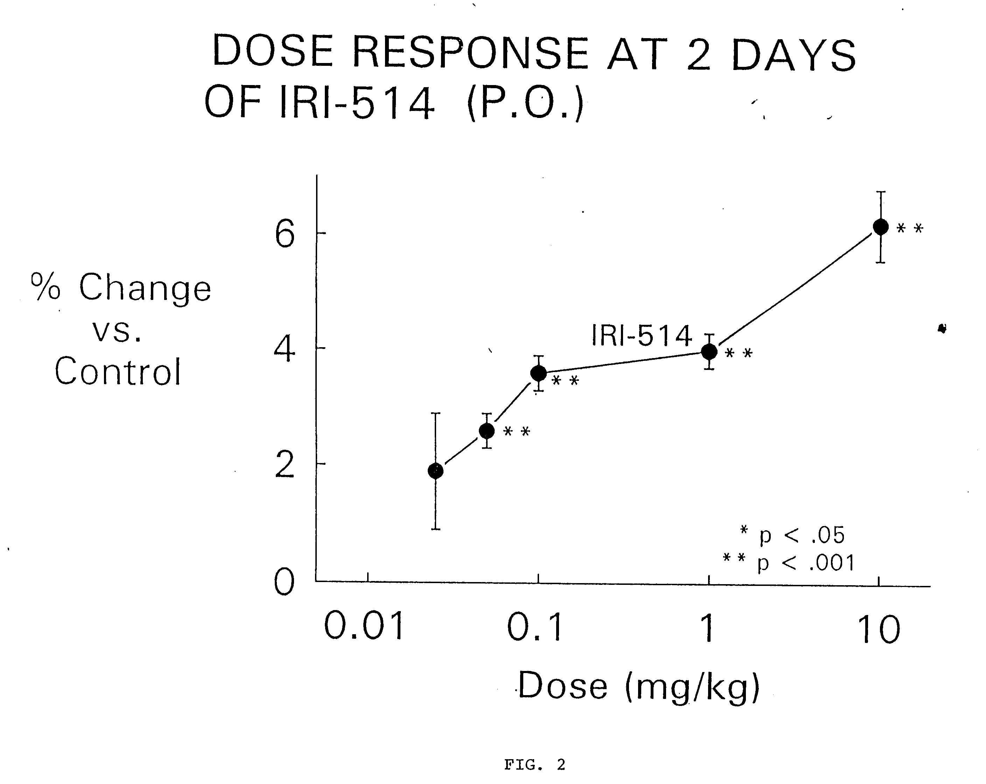

With supramaximal nerve stimulation, at 30 impulses per second, the height of the tenth muscle action potential was expressed as a percentage of the first. P-values for testing statistical differences between the controls and the testing compounds were calculated by Dunnett's two-tailed t-test. Figure 2 shows a dose-response effect of oral IRI-514 on neuromuscular transmission, with a threshold dose of 0.05 mg/kg.

Example 19 - Regulation of Anti-CD3 Stimulated T Suppressor Cells

Female NZB x NZW F1 mice were purchased from Jackson Laboratories (Bar Harbour, ME) and housed in shoebox cages in contact bedding, six animals per cage. Food and water were supplied ad libitum. Animals were humanely sacrificed with CO$_2$ at the conclusion of the treatment period.

Mice were treated with test peptides prior to sacrifice on one of two treatment protocols. In protocol A, mice received peptides dissolved in bacteriostatic water for injection, 1 mg/kg subcutaneously once per week for four weeks starting at four weeks of age. In protocol B, seven week old mice were given four injections of 1 mg/kg subcutaneously on four consecutive days. All animals were sacrificed for assay of T suppressor cell activity on the day following the last injection.

Spleens were aseptically removed, single cell suspensions prepared from each individual animal by passage through 60 mesh screen, and suspended in tissue culture medium consisting of RPMI 1640 with 25 mM Hepes buffer, 10% fetal bovine serum, 2% sodium pyruvate, 1% NEN non-essential amino acids, 1% L-glutamine (all from Gibco, Grand Island, NY), 1% β-mercaptoethanol (Sigma, St. Louis, MO), and 0.05% gentamicin (Flow, McLean, VA).

Suppressor cells for mitogen stimulated T cell proliferative responses were generated on 35 mm tissue culture wells (Corning, Corning, NY), coated with anti-CD3 (Clone 145-2C11, Boehringer Mannheim, Indianapolis, IN), diluted in borate buffered saline pH 8.5 at a concentration of 0.5 μg/ml. These plates were incubated at room temperature for 4 hours and washed three times with Hanks balanced salt solution before addition of cells. Two ml of splenocytes from individual animals at $5 \times 10^6$ cells/ml were added, and the cultures incubated for 48 hours in 5% $CO_2$ at 37°C. The stimulated cultures were harvested and the cells adjusted to $1.25 \times 10^6$ cells/ml for addition to the suppression assay culture at 25% of the responding cell number. Suppression of T cell proliferation was measured in 48 hour mitogen stimulated cultures (0.25 $\mu$g/ml anti-CD3, Boehringer Mannheim) containing $5 \times 10^5$ fresh syngeneic splenocytes and $1.25 \times 10^5$ test cells in triplicate cultures in 96 well plates. The wells were pulsed during the last seven hours with 1 $\mu$Ci [$^3$H] thymidine (NEN Research Products, Chadds Ford, PA), harvested on filtermats (Pharmacia, Turku, Finland) and counted on an LKB 1205 Betaplate liquid scintillation counter.

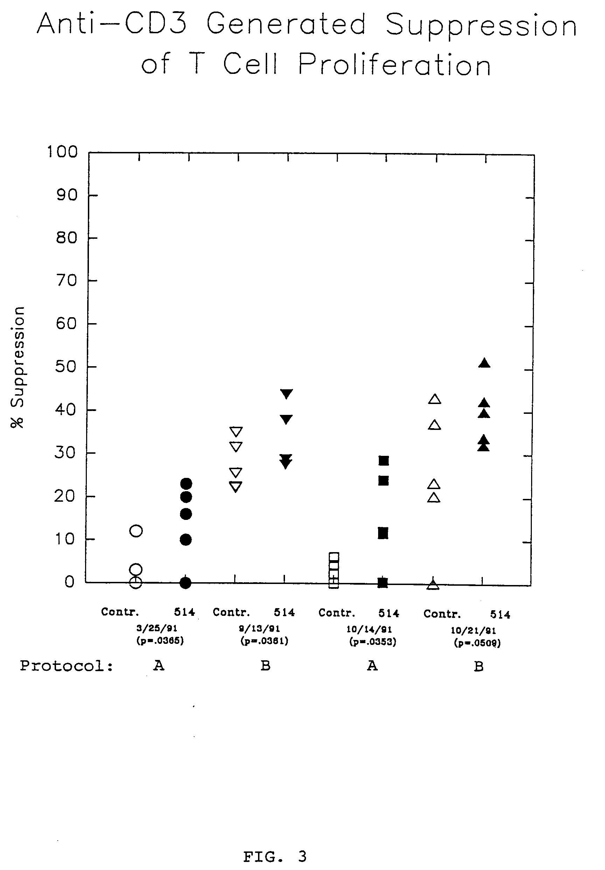

Figure 3 summarizes four experiments which show that cells derived from IRI-514 (Acetyl-Arg-Pro-Asp-Pro-Phe-NH$_2$) dosed animals, either daily for 4 days (Protocol B) or once per week for 4 weeks (Protocol A), were more effective at generating suppressor cells in this assay than cells from vehicle (placebo) treated animals. Each symbol represents a different group of animals. Open symbols are controls. Black symbols are IRI-514-treated animals.

## Example 20 - Effects of IRI-514 on the Behavioral Response after Social Stress-Induced Anxiety in Rats

Exposure to social stress in mammals results in the activation of the hypothalamic-pituitary-adrenal (HPA) axis, and in changes in emotionality and increased anxiety. This can be studied in rats by testing the behavior of stressed animals in an elevated plus maze. The maze consists of two arms with 40 cm high walls (the closed arms), and two arms with no walls (the open arms). Whereas normal, unstressed animals will spend comparable time exploring each of the arms of the maze, animals that exhibit increased anxiety and changes in emotionality spend significantly less time exploring the open arms of the maze.

In the resident-intruder paradigm of social stress, an intruder male rat is placed in the home cage of an aggressive, resident male. This results in an interaction which generally establishes the dominance of the resident rat. The defeated animals demonstrate elevated levels of HPA axis-derived hormones, as well as increased anxiety as determined in the plus-maze test.

In these studies, 15-17 intruder rats were treated with IRI-514 at 1 mg/kg, or with vehicle (normal saline) subcutaneously, 48 hours prior to being placed in the cage of a resident male rat. The intruder rats were exposed to the resulting interaction for 30 minutes. Physical harm was avoided by protecting the intruder in a plexiglass cage, thus providing a model for relatively pure psychological stress. Individual IRI-514- and vehicle-treated animals were then placed on the center of the elevated plus maze, and allowed to explore all four arms of the maze for 5 minutes. IRI-514- and vehicle-treated animals that were not exposed to a resident rat were also tested as controls. Computer automated measures of the duration of the exploration of each arm of the maze by each animal were recorded.

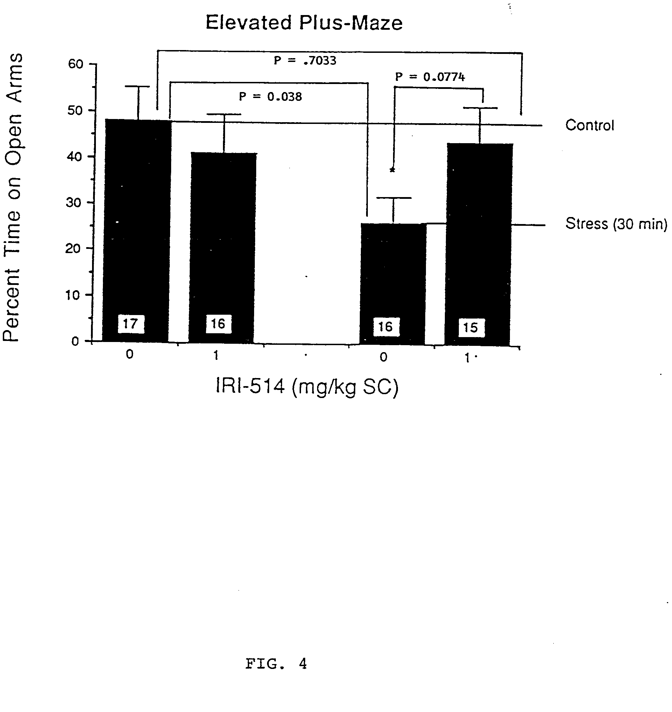

As shown in Figure 4, vehicle-treated, and IRI-514-treated, control (unstressed) animals spent, respectively, 47.9±7.3% and 41.3±8.3 (mean±sem) of the time exploring the open arms of the maze. Vehicle-treated rats that had been exposed to social stress, in contrast, spent significantly less time in the open arms of the maze (26.4±5.5%; mean±sem; p=0.038). More importantly, the rats that received IRI-514 prior to being exposed to the social stress spent comparable time in the open arms of the maze as did the control, unstressed rats (43.8±7.9; mean±sem). The overall activity of the rats was similar in all groups.

These results strongly suggest that IRI-514 has the capacity to prevent or normalize the changes in the behavioral responses that are associated with increased anxiety and altered emotionality upon exposure to psychological (social) stress.

## Example 21 - Effects of IRI-514 on the Hormones of the HPA Axis after Social Stress-Induced Anxiety in Rats

Exposure to social stress in mammals results in the activation of the HPA axis and in elevated levels of circulating corticotropin (ACTH) and corticosteroids (COR).

In the resident-intruder paradigm of social stress, an intruder male rat is placed in the home cage of an agressive, resident male. This results in an interaction which generally establishes the dominance of the resident rat. The defeated animals consistently demonstrate elevated levels of HPA axis-derived hormones, and increased anxiety.

In these studies, 13-16 intruder rats were fitted with chronically indwelling jugular catheters in order to facilitate the collection of blood samples, and allowed to recover. The animals were then injected subcutaneously with either IRI-514 at 1 mg/kg, or with an equal volume of vehicle (normal saline; control rats), 24 hours prior to being placed in the cage of a resident male rat. A sample of blood was collected immediately prior to exposure to the stressor, in order to determine basal levels of plasma ACTH and COR. The intruder rats were then exposed to the resident male and to the resulting agonistic interaction for 30 minutes. Physical harm was avoided by protecting the intruder in a plexiglass cage, thus providing a model for relatively pure psychological stress. Blood samples were collected at 10 minutes and at 20 minutes into the encounter, and the levels of ACTH and COR were determined.

Table 1 summarizes the results of these experiments. Shown are the geometric means and confidence intervals (C.I.) of the levels of ACTH and COR for the control or IRI-514-treated rats measured prior to, and 10 and 20 minutes after exposure to the stress. Basal levels of ACTH were similar for control and IRI-514-treated animals (P = 0.2817). In the control rats, the peripheral levels of ACTH rose from 3.4 pg/mL to 56.8 pg/mL, at 10 minutes after stress, and remained elevated after 20 minutes of the exposure to the social paradigm. More importantly, after 10 minutes of stress exposure, significantly lower levels (P = 0.0370) of ACTH were found in the IRI-514-treated rats (28.2 pg/mL) as compared to the controls. ACTH levels were also lower in the IRI-514-treated animals (25.2 pg/mL) than in the control rats (52.5 pg/mL) when measured 20 minutes after exposure to the stressor. This latter difference, however, did not reach statistical significance (P = 0.0788). No differences were observed between control and IRI-514-treated rats in the levels of COR prior to or after exposure to the stressor.

## Table 1

### Vehicle (Placebo)

| Hormone | Time (Min.) | N | Geometric Mean | Lower C.I. | Upper C.I. | p-Value |
|---------|-------------|----|----------------|------------|------------|---------|
| ACTH | Baseline | 14 | 3.4 | 1.6 | 5.2 | 0.2817 |
| | 10 | 14 | 56.8 | 40.5 | 73.1 | 0.0370 |
| | 20 | 14 | 52.5 | 35.2 | 69.8 | 0.0788 |
| Cortico-sterone | Baseline | 13 | 35.5 | 27.9 | 43.1 | 0.1906 |
| | 10 | 13 | 245.7 | 210.7 | 280.7 | 0.7022 |
| | 20 | 13 | 229.2 | 175.7 | 282.7 | 0.9687 |

### IRI-514 (1mg/kg)

| Hormone | Time (Min.) | N | Geometric Mean | Lower C.I. | Upper C.I. | p-Value |
|---------|-------------|----|----------------|------------|------------|---------|
| ACTH | Baseline | 16 | 1.5 | 0.7 | 2.3 | 0.2817 |
| | 10 | 16 | 28.2 | 23.1 | 33.3 | 0.0370 |
| | 20 | 16 | 25.2 | 17.7 | 32.7 | 0.0788 |
| Cortico-sterone | Baseline | 15 | 46.5 | 36.5 | 56.5 | 0.1906 |
| | 10 | 15 | 230.9 | 210.9 | 250.9 | 0.7022 |
| | 20 | 13 | 221.5 | 189.5 | 253.5 | 0.9687 |

The p-Value is a randomized block design (two-tailed) based on LOG transformed data.
Lower C.I. = geometric mean - STE
Upper C.I. = geometric mean + STE.

These results strongly suggest that IRI-514 has the capacity to suppress or modulate the elevation of HPA-axis derived hormones that are associated with increased anxiety and altered emotionality upon exposure to psychological (social) stress.

Example 22 - In Vivo Stability of IRI-514

This study was performed to investigate the disposition and pharmacokinetics of total radioactivity following a single intravenous (IV) administration of $^{14}$C-IRI-514 to rats. Peptide IRI-514 was synthesized as described in Example 1 above, incorporating $^{14}$C-Arg to enable tracking of the compound by detection of radioactive emissions following administration to animals. Plasma and urinary radioactivity were analyzed to determine the disposition characteristics of $^{14}$C-IRI-514-related material at two dose levels, 0.1 and 10 mg IRI-514/kg.

Male rats (three for the 0.1 mg/kg and two for the 10 mg/kg dose level) were catheterized via their jugular vein, carotid artery, and the urinary bladder. Animals were maintained with a 5% dextrose infusion via a jugular vein catheter and the urinary bladder was continuously irrigated with sterile water to stimulate frequent voiding. The dilute urine was collected using a penile catheter and fraction collector. Each animal received 10 μCi $^{14}$C-IRI-514 with unlabeled IRI-514 at a total dose level of either 0.1 or 10 mg/kg through the jugular vein catheter. Blood samples were collected from the carotid artery catheter pre-dose and at 1, 5, 10, 20, 40, 80 minutes and 2, 4, 6, and 24 hours post dose. The dilute urine was collected for the time intervals 0-5, 5-10, 10-20, 20-40, 40-80, 80-120 minutes and every 15 minutes thereafter until 24 hours after dosing. Plasma and urine samples that exhibited sufficient radioactivity were assayed by high pressure liquid chromatography (HPLC) for parent $^{14}$C-IRI-514 content to estimate the extent of metabolism.

Results after both dose levels indicated a $t^{1/2}$ (elimination half-life) ranging between 25.3 to 28 minutes determined by plasma total radioactivity data observed in the samples taken between 10 minutes and 2 hours after dose administration. The area under the plasma concentration versus time curve (AUC) 0-24 hours was proportional to the dose administered. The AUC following the low dose was 0.162 and 0.164 μg-equivalents x hour/mL and the AUC after the high dose was 20.11 and 22.23 μg-equivalents x hour/mL. HPLC analysis of plasma samples obtained 0-40 minutes after each dose administration indicated limited metabolism at both dose levels. Approximately 87.9 to 96.4% of the total radioactivity in the 1 to 2 minute sample was parent peptide; 44-61.9% of the total radioactivity in the 40-minute sample was parent peptide.

Analysis of the urine data from both dose levels indicated that between 38.8 and 67.1% was eliminated within 40 minutes; between 73.9 and 82.9% was eliminated within 2 hours; and between 86.2 and 94% of parent and metabolites were eliminated in 24 hours of dosing. Between 62.5 and 68.3% of the total radioactivity eliminated in the first 5 minutes was shown to be parent peptide by HPLC analysis. In the 40-80 minute post-dose collection sample 5.4-18.6% of the total radioactivity eliminated was identified as parent peptide.

It was concluded that levels of total radioactivity, IRI-514 and its metabolites, declined rapidly in plasma following IV administration of $^{14}$C-IRI-514 due to renal clearance. The disposition and the pharmacokinetics of IRI-514 following IV administration appear to be independent of the dose (either 0.1 or 10 mg/kg) as determined by plasma $t^{1/2}$, AUC and urinary excretion data comparison. Finally, there is limited metabolism of the peptide while in circulation.

B. Stability conferred by Pro in position 4 of Peptide

Acetyl-Arg-Pro-Asp-Val-Phe-NH$_2$ and Acetyl-Arg-Pro-Asp-Pro-Phe-NH$_2$ [IRI-514] were synthesized by the methods described above, incorporating $^{14}$C-Arg to enable tracking of the compound by detection of radioactive emissions following administration to animals. Following intravenous administration to rats, according to the techiques described above in part A, more than 90% of administered radioactivity was detected in urine over six (6) hours for both compounds.

The urine was then studied by HPLC in a system that was shown to separate intact parent compound from its breakdown products. For Acetyl-Arg-Pro-Asp-Val-Phe-NH$_2$ approximately 25% of the urinary radioactivity was associated with parent compound whereas the Acetyl-Arg-Pro-Asp-Pro-Phe-NH$_2$ 64% of radioactivity was found to be associated with parent compound. Thus the Pro substitution for Val at position 4 conferred enhanced stability of the compound during passage in the body and urinary elimination after intravenous administration.

Numerous modifications and variations of the present invention are included in the above-identified specification and are expected to be obvious to one of skill in the art. For example, peptides other than those exemplified which fall within the above formula may be produced by a variety of methods. The peptides may be tested in the above assays without undue experimentation by one of skill in the art, given this disclosure. Such modifications and alterations to the compositions and processes of the present invention are believed to be encompassed in the scope of the claims appended hereto.

**Claims**

1. A pentapeptide having the formula:

$$R^1-V-W-X-Y-Z-R^2$$

or a pharmaceutically acceptable acid- or base-addition salt thereof, wherein

$R^1$ is hydrogen or a $C_1$ to $C_{10}$ lower alkyl or alkanoyl;

V is Arg;

W is Pro, dehydro-Pro or hydroxy-Pro;

X is Asp, Ser, Thr, Ala, Asn, Glu, or Gln;

Y is Pro, dehydro-Pro, or hydroxy-Pro;

Z is Phe or Tyr, optionally substituted with one or more halogen, nitro or hydroxyl group; and

$R^2$ is OH or $NR^3R^4$,

wherein $R^3$ and $R^4$ are each independently selected from the group consisting of H, a straight chain or branched alkyl or alkenyl having 1 to 6 carbon atoms, optionally substituted with an aryl group or aryl substituted with either a halogen or a straight chain, a branched alkyl or alkenyl having 1 to 6 carbon atoms, or $R^3$ and $R^4$ together comprise a cyclic methylene group of 3 to 7 carbon atoms, and

wherein all of V, W, X, Y and Z are L-amino acids or optionally one or more of V, W, X, Y and Z are D amino acids.

2. A pharmaceutical composition comprising a therapeutically effective amount of at least one pentapeptide of claim 1 in a pharmaceutically acceptable formulation.

3. The composition according to claim 2 which is suitable for oral administration.

4. A pentapeptide selected from the group consisting of:

Acetyl-Arg-Pro-Asp-Pro-Phe-NH$_2$

Acetyl-Arg-Pro-Asp-Pro-Tyr-NH$_2$

Acetyl-Arg-Pro-Asn-Pro-Phe-NH$_2$

Acetyl-Arg-Pro-Asn-Pro-Tyr-NH$_2$

Acetyl-Arg-Pro-Ala-Pro-Phe-NH$_2$

Acetyl-Arg-Pro-Ala-Pro-Tyr-NH$_2$

Acetyl-Arg-Pro-Glu-Pro-Tyr-NH$_2$

Acetyl-Arg-Pro-Ser-Pro-Phe-NH$_2$

Acetyl-Arg-Pro-Thr-Pro-Phe-NH$_2$

Acetyl-Arg-Pro-Thr-Pro-Tyr-NH$_2$

Acetyl-Arg-Pro-Ser-Pro-Tyr-NH$_2$

Acetyl-Arg-Pro-Asp-Pro-pClPhe-NH$_2$

Decanoyl-Arg-Pro-Asp-Pro-Phe-NH$_2$

Acetyl-Arg-Pro-Glu-Pro-Phe-NH$_2$

Butyryl-Arg-Pro-Asn-Pro-Phe-NH$_2$

5. A method for treating a subject with a physical condition characterized by a disorder of the immune system comprising administering to said subject a therapeutically effective amount of at least one pentapeptide having the formula:

$$R^1-V-W-X-Y-Z-R^2$$

or a pharmaceutically acceptable acid- or base-addition salt thereof, wherein

$R^1$ is hydrogen or a $C_1$ to $C_{10}$ lower alkyl or alkanoyl;

V is Arg;

W is Pro, dehydro-Pro or hydroxy-Pro;

X is Asp, Ser, Thr, Ala, Asn, Glu, or Gln;

Y is Pro, dehydro-Pro, or hydroxy-Pro;

Z is Phe or Tyr, optionally substituted with one or more halogen, nitro or hydroxyl group; and

$R^2$ is OH or $NR^3R^4$,

wherein $R^3$ and $R^4$ are each independently selected from the group consisting of H, a straight chain or branched alkyl or alkenyl having 1 to 6 carbon atoms, optionally substituted with an aryl group or aryl substituted with either a halogen or a straight chain, a branched alkyl or alkenyl having 1 to 6 carbon atoms, or R³ and R⁴ together comprise a cyclic methylene group of 3 to 7 carbon atoms, and

wherein all of V, W, X, Y and Z are L-amino acids or optionally one or more of V, W, X, Y and Z are D amino acids.

6. A method for treating a subject with a physical condition characterized by a disorder of the central nervous system comprising administering to said subject a therapeutically effective amount of at least one pentapeptide having the formula:

$$R^1\text{-}V\text{-}W\text{-}X\text{-}Y\text{-}Z\text{-}R^2$$

or a pharmaceutically acceptable acid- or base-addition salt thereof, wherein

$R^1$ is hydrogen or a $C_1$ to $C_{10}$ lower alkyl or alkanoyl;

V is Arg;

W is Pro, dehydro-Pro or hydroxy-Pro;

X is Asp, Ser, Thr, Ala, Asn, Glu, or Gln;

Y is Pro, dehydro-Pro, or hydroxy-Pro;

Z is Phe or Tyr, optionally substituted with one or more halogen, nitro or hydroxyl group; and

$R^2$ is OH or $NR^3R^4$,

wherein $R^3$ and $R^4$ are each independently selected from the group consisting of H, a straight chain or branched alkyl or alkenyl having 1 to 6 carbon atoms, optionally substituted with an aryl group or aryl substituted with either a halogen or a straight chain, a branched alkyl or alkenyl having 1 to 6 carbon atoms, or $R^3$ and $R^4$ together comprise a cyclic methylene group of 3 to 7 carbon atoms, and

wherein all of V, W, X, Y and Z are L-amino acids or optionally one or more of V, W, X, Y and Z are D amino acids.

7. A method for preparing a pentapeptide having the formula:

$$R^1\text{-}V\text{-}W\text{-}X\text{-}Y\text{-}Z\text{-}R^2$$

or a pharmaceutically acceptable acid- or base-addition salt thereof, wherein

$R^1$ is hydrogen or a $C_1$ to $C_{10}$ lower alkyl or alkanoyl;

V is Arg;

W is Pro, dehydro-Pro or hydroxy-Pro;

X is Asp, Ser, Thr, Ala, Asn, Glu, or Gln;

Y is Pro, dehydro-Pro, or hydroxy-Pro;

Z is Phe or Tyr, optionally substituted with one or more halogen, nitro or hydroxyl group; and

$R^2$ is OH or $NR^3R^4$,

wherein $R^3$ and $R^4$ are each independently selected from the group consisting of H, a straight chain or branched alkyl or alkenyl having 1 to 6 carbon atoms, optionally substituted with an aryl group or aryl substituted with either a halogen or a straight chain, a branched alkyl or alkenyl having 1 to 6 carbon atoms, or $R^3$ and $R^4$ together comprise a cyclic methylene group of 3 to 7 carbon atoms, and

wherein all of V, W, X, Y and Z are L-amino acids or optionally one or more of V, W, X, Y and Z are D amino acids;

said method comprising solid phase synthesis.

8. A method for preparing a pentapeptide having the formula:

$$R^1\text{-}V\text{-}W\text{-}X\text{-}Y\text{-}Z\text{-}R^2$$

or a pharmaceutically acceptable acid- or base-addition salt thereof, wherein

$R^1$ is hydrogen or a $C_1$ to $C_{10}$ lower alkyl or alkanoyl;

V is Arg;

W is Pro, dehydro-Pro or hydroxy-Pro;

X is Asp, Ser, Thr, Ala, Asn, Glu, or Gln;

Y is Pro, dehydro-Pro, or hydroxy-Pro;

Z is Phe or Tyr, optionally substituted with one or more halogen, nitro or hydroxyl group; and

$R^2$ is OH or $NR^3R^4$,

wherein $R^3$ and $R^4$ are each independently selected from the group consisting of H, a straight chain or branched alkyl or alkenyl having 1 to 6 carbon atoms, optionally substituted with an aryl group or aryl substituted with either a halogen or a straight chain, a branched alkyl or alkenyl having 1 to 6 carbon atoms, or $R^3$ and $R^4$ together comprise a cyclic methylene group of 3 to 7 carbon atoms, and

wherein all of V, W, X, Y and Z are L-amino acids or optionally one or more of V, W, X, Y and Z are D amino acids;

said method comprising solution phase synthesis.

9. The method according to claim 8 wherein said pentapeptide is selected from the group consisting of:

Acetyl-Arg-Pro-Asp-Pro-Phe-NH$_2$,
Acetyl-Arg-Pro-Asp-Pro-Tyr-NH$_2$,
Acetyl-Arg-Pro-Asn-Pro-Phe-NH$_2$,
Acetyl-Arg-Pro-Asn-Pro-Tyr-NH$_2$,
Acetyl-Arg-Pro-Glu-Pro-Tyr-NH$_2$,
Acetyl-Arg-Pro-Ser-Pro-Phe-NH$_2$,
Acetyl-Arg-Pro-Thr-Pro-Phe-NH$_2$,
Acetyl-Arg-Pro-Thr-Pro-Tyr-NH$_2$,
Acetyl-Arg-Pro-Ser-Pro-Tyr-NH$_2$,
Acetyl-Arg-Pro-Asp-Pro-pClPhe-NH$_2$,
Decanoyl-Arg-Pro-Asp-Pro-Phe-NH$_2$,
Acetyl-Arg-Pro-Glu-Pro-Phe-NH$_2$, and
Butyryl-Arg-Pro-Asn-Pro-Phe-NH$_2$.

FIG. 1

ENZYMATIC STABILITY OF TP-5 AND PEPTIDE #514

DOSE RESPONSE AT 2 DAYS
OF IRI-514 (P.O.)

FIG. 2

Anti—CD3 Generated Suppression
of T Cell Proliferation

FIG. 3

FIG. 4

European Patent
Office

**PARTIAL EUROPEAN SEARCH REPORT**
which under Rule 45 of the European Patent Convention
shall be considered, for the purposes of subsequent
proceedings, as the European search report

Application number

| DOCUMENTS CONSIDERED TO BE RELEVANT | | | EP 92305000.9 |
|---|---|---|---|

| Category | Citation of document with indication, where appropriate, of relevant passages | Relevant to claim | CLASSIFICATION OF THE APPLICATION (Int Cl⁵) |
|---|---|---|---|
| X | <u>DD - A - 263 994</u><br>(MARTIN-LUTHER-UNIV.<br>HALLE-WITTENBG.)<br>* Claim 1, pentin(II) *<br>-- | 1 | C 07 K 7/06<br>C 07 K 1/02<br>C 07 K 1/04<br>A 61 K 37/02 |
| A | CHEMICAL ABSTRACTS, vol. 105,<br>no. 1, July 07, 1986<br>Columbus, Ohio, USA<br>E. RAJNAVOLGYI et al. "The<br>influence of new thymopoietin<br>derivatives on the immune<br>response of inbred mice.",<br>page 74, column 1,<br>abstract-no. 800x<br>     & Int. J. Immunopharmacol.<br>     1986, 8(2), 167-77<br>-- | 1-4,<br>7-9 | |
| P,A | CHEMICAL ABSTRACTS, vol. 115,<br>no. 13, September 30, 1991<br>Columbus, Ohio, USA<br>ROZSA KASSAI TANCZOS et al.<br>"Submolecular acid-base pro-<br>perties of bioligands.<br>III. Microspeciation of | 1-4,<br>7-9 | TECHNICAL FIELDS SEARCHED (Int Cl⁵)<br><br>C 07 K 7/00<br>C 07 K 1/00<br>C 07 H 21/00<br>A 61 K 37/00 |

**INCOMPLETE SEARCH**

The Search Division considers that the present European patent application does not comply with
the provisions of the European Patent Convention to such an extent that it is not possible to carry
out a meaningful search into the state of the art on the basis of some of the claims.

Claims searched completely:    1-4, 7-9

Claims searched incompletely:    −

Claims not searched:    5,6

Reason for the limitation of the search:

Art. 52(4) EPC

Method for treatment of the human or animal body by
therapy

| Place of search | Date of completion of the search | Examiner |
|---|---|---|
| VIENNA | 25-08-1992 | SCHARF |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1505.1 03.82

European Patent
Office

**PARTIAL EUROPEAN SEARCH REPORT**

| | DOCUMENTS CONSIDERED TO BE RELEVANT | | CLASSIFICATION OF THE APPLICATION (Int. Cl.4) 5 |
|---|---|---|---|
| Category | Citation of document with indication, where appropriate, of relevant passages | Relevant to claim | |
| | thymopoietin-type tetra-peptides and their derivatives.", page 1053, column 1, abstract-no. 136 769h & Mag. Kem. Foly. 1991, 97(4), 149-56 -- | | |
| P,A | CHEMICAL ABSTRACTS, vol. 115, no. 17, October 28, 1991 Columbus, Ohio, USA BELA NOSZAL et al. "Acid-base properties of thymopoietin--type tri- and tetrapeptides and their derivatives.", page 992, column 2, abstract-no. 183 935u & Int. J. Pept. Protein Res. 1991, 38(2), 139-45 -- | 1-4, 7-9 | TECHNICAL FIELDS SEARCHED (Int. Cl.K) 5 |
| P,A | CHEMICAL ABSTRACTS, vol. 115, no. 19, November 11, 1991 Columbus, Ohio, USA HUBERT KALBACHER et al. "Acid labile protection of histidine and arginine in SPPS using Adpoc deri-vatives.", page 1079, column 2, abstract-no. 208 510s & Pept. 1990, Proc. Eur. Pept. Symp., 21st 1990 (Pub. 1991), 31-3 ---- | 1-4, 7-9 | |

EPO Form 1505.3    06.78